# EUROPÄISCHE PATENTANMELDUNG

(11) **EP 1 468 664 A1**
(43) Veröffentlichungstag der Anmeldung: **20.10.2004**
(21) Anmeldenummer: 04007774.5
(22) Anmeldetag: 31.03.2004
(51) Int. Cl.: A61K 7/043

(54) **Nageldekorschicht mit mindestens einer UV-Gel-Schicht und mit Körperpuder**

(30) Priorität: 15.04.2003 DE 20306086 U
(71) Anmelder: Olavarria-Navarro, Christiane, 35394 Giessen (DE)
(72) Erfinder: Olavarria-Navarro, Christiane, 35394 Giessen (DE)
(74) Vertreter: Lösch, Christoph

(57) **Zusammenfassung**

Die Neuerung betrifft eine Nageldekorschicht mit mindestens einer UV-Gel-Schicht zur Erzeugung einer glänzenden Oberfläche mit eingerührtem oder eingefärbtem Körperpuder zur Erzeugung von Farbeffekten und zur besseren Aushärtung der Nageldekorschicht.

## Beschreibung

Die Neuerung betrifft eine Nageldekorschicht zur Anbringung auf Kunstnägeln oder Gel-verstärkten Naturnägeln.

Der Neuerung liegt die Aufgabe zugrunde, eine Nageldekorschicht mit besonders langer Haltbarkeit anzubieten.

Diese Aufgabe wird durch eine Nageldekorschicht mit den Merkmalen des Schutzanspruchs 1 gelöst. Vorteilhafte Ausführungsformen der Nageldekorschicht sind in den Unteransprüchen 2 - 10 beschrieben.

Bei der neuerungsgemäßen Nageldekorschicht ist mindestens eine UV-Gel-Schicht zur Erzeugung einer glättenden Oberfläche mit eingerührtem odereingefärbtem Körperpuder zur Erzeugung von Farbeffekten und zur besseren Aushärtung der Nageldekorschicht vorgesehen. Durch Verwendung einer derartigen UV-Gel-Schicht mit eingebrachtem Körperpuder entsteht überraschenderweise eine Nageldekorschicht mit besonders langer Haltbarkeit von etwa 4 - 6 Wochen.

Die neuerungsgemäße Nageldekorschicht härtet innerhalb kurzer Zeit (etwa 90 - 120 sec) aus, so daß auch keine langen Trocknungszeiten bei der Anbringung der Nageldekorschicht für den Kunden für das Nagelstudio anfallen. Damit kann die Nageldekorschicht innerhalb kurzer Zeit aufgebracht werden und besitzt die beschriebene lange Haltbarkeit sowie eine sehr hohe Abriebsfestigkeit.

Bei der neuerungsgemäßen Nageldekorschicht können auch zusätzliche Elemente, wie z.B. Straßsteinchen, Einlegemotive, Plattgold, Plattsilber, Konfetti, Glitzerpuder und/oder Glitzerstaub aufgenommen und durch die extrem kurzen Trocknungszeiten innerhalb kürzester Zeit fixiert werden. Bislang übliche lange Trocknungszeiten, die sowohl für das Nagelstudio als auch die behandelnde Person in Kauf genommen werden mußten, fallen nun nicht mehr an.

Die neuerungsgemäße Nageldekorschicht besitzt zum einen ein UV-Glanz-Gel, welches die Inhaltsstoffe Hydroxyfunct. Methacrylate, Urethane Acrylate, Aliphphatic-Metacryl-Oligomere, Methacrylfunc. Phosphorylester, Methacrylfunc. Oligamere, Siliciumdioxyde, Activatoren und/oder Inhibitoren aufweisen kann.

Der in das UV-Glanz-Gel eingerührte oder eingefärbte Körperpuder kann die Bestandteile Talc, PVP, Peg-150Fragrance (Parfum) sowie Titanium Dioxide Cl 77891, Iron Oxide red Cl 77491, Iron Oxide yellow Cl 77492, Iron Oxide black Cl 77499, Ultramarine blue Cl 77007, Carmine Cl 75470, DC red 36 Cl 12085, Pigmente yellow 1 Cl 11680, Barium Sulfate Cl 77120, Alizarin Cl 58000, Pigmente green 7 Cl 74260, Food blue1 Cl 73015:1, Mica Cl 77019, Ferric Ferrocyanide Cl 77510 und/oder FDC yellow 5 Cl 10140:1 besitzen.

Nach einer weiteren vorteilhaften Ausführungsform kann zur Erzielung eines Metalliceffekts zusätzlich Silber- oder Bronzepuder eingemischt werden. Dabei kann z.B. Kryolan-Transparentpuder eingemischt werden.

Insgesamt ermöglicht es die neuerungsgemäße Nageldekorschicht dauerhafte Designs auf Kunst- oder Naturnägeln anzubringen und bietet ferner dabei eine extrem kurze Trocknungszeit. Somit erhöht sich die Wirtschaftlichkeit für ein Nagelstudio und der Behandlungskomfort für die jeweilige bediente Person.

Die Neuerung ist anhand eines Ausführungsbeispiels in einer Zeichnungsfigur näher erläutert.

Die Zeichnungsfigur zeigt einen möglichen Schichtaufbau mit einer unteren Naturnagelschicht 1 auf welcher die neuerungsgemäße Nageldekorschicht 2 angebracht ist. Zur Abdeckung und zum Schutz kann eine weitere Deckschicht 3 auf der Nageldekorschicht 2 angebracht sein. In der Nageldekorschicht 2 können Zusatzelemente 4, wie z.B. Straßsteinchen aufgenommen sein.

### BEZUGSZEICHEN

- 1: Naturnagelschicht
- 2: Nageldekorschicht
- 3: Deckschicht
- 4: Zusatzelemente

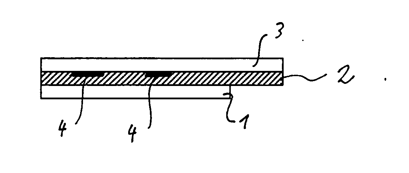

## Patentansprüche

1. Nageldekorschicht mit mindestens einer UV-Gel-Schicht zur Erzeugung einer glänzenden Oberfläche mit eingerührtem oder eingefärbtem Körperpuder zur Erzeugung von Farbeffekten und zur besseren Aushärtung der Nageldekorschicht.

2. Nageldekorschicht nach Anspruch 1 , wobei in der Nageldekorschicht Straßsteinchen aufgenommen sind.

3. Nageldekorschicht nach einem der vorhergehenden Ansprüche, wobei in der Nageldekorschicht Einlegemotive aufgenommen sind.

4. Nageldekorschicht nach einem der vorhergehenden Ansprüche, wobei in der Nageldekorschicht Blattgold aufgenommen ist.

5. Nageldekorschicht nach einem der vorhergehenden Ansprüche, wobei in der Nageldekorschicht Blattsilber aufgenommen ist.

6. Nageldekorschicht nach einem der vorhergehenden Ansprüche, wobei in der Nageldekorschicht Konfetti aufgenommen ist.

7. Nageldekorschicht nach einem der vorhergehenden Ansprüche, wobei in der Nageldekorschicht Glitzerpuder aufgenommen ist.

8. Nageldekorschicht nach einem der vorhergehenden Ansprüche, wobei in der Nageldekorschicht Glitzerstaub aufgenommen ist.

9. Nageldekorschicht nach einem der vorhergehenden Ansprüche, wobei zur Erzielung eines Metallic-Effekts zusätzlich Silber- oder Bronzepuder eingemischt ist.

10. Nageldekorpuder nach Anspruch 9, wobei Kryolan-Transparentpuder eingemischt ist.
